# EUROPEAN PATENT SPECIFICATION

(11) **EP 2 542 916 B1**
(45) Date of publication and mention of the grant of the patent: **15.02.2017**
(21) Application number: 11713062.5
(22) Date of filing: 28.02.2011
(51) Int. Cl.: G01T 1/164, G01T 1/161

(54) **SCINTIGRAPHIC GONIOMETRIC PROBE**
SZINTIGRAPHISCHE WINKELMESSSONDE
SONDE GONIOMÉTRIQUE SCINTIGRAPHIQUE

(30) Priority: 02.03.2010 IT RM20100082
(43) Date of publication of application: 09.01.2013
(73) Proprietor: Università degli Studi di Roma "La Sapienza", 00185 Roma (IT)
(72) Inventor: PANI, Roberto, I-00185 Roma (IT)
(74) Representative: Leone, Mario
(86) International application number: PCT/IB2011/050851
(87) International publication number: WO 2011/107930

(56) References cited:
- EP-A1- 1 596 223
- US-A- 3 539 806
- US-B1- 6 643 538

## Description

The present invention relates to a scintigraphic goniometric probe of the type used to detect the emission of a radiation and its origin direction, in order to identify a radiation source, in particular in a scintigraphic examination.

This type of examination is generally performed by injecting a radioactive tracer in the human organism so that it may accumulate at a specific tissue. In particular, this examination can be used to identify the so-called sentinel lymph node, that is the first lymph node which is reached by possible metastasis starting from malignant tumours spreading through the lymphatic route.

Therefore, generally the detection of the anomalous tumoral mass is performed by detecting a ionizing radiation, X- or gamma rays, emitted by an accumulation of said substance in the tissue subjected to examination. Said radiation is emitted, in direct or indirect way, during the decay of the radioisotopes used to mark the radiomedicine.

The probe is then used during the scintigraphic examination to identify the exact position of the sentinel lymph node, so as to be able to intervene by performing a biopsy.

However, it is meant that the probe, as instrument to identify the origin of a radiation, can be used with any type or radioactive source.

The operation of a scintigraphic probe generally is based upon the capability of some types of crystals to generate photons of visible light when hit by the radiation coming from the source.

These photons are highlighted upon using photomultipliers and they are transformed in electric pulses.

The number of events detected in the time unit is proportional to the radioisotope concentration inside the measuring cone of the instrument. The detection of the high emission sites takes place by comparing the countings performed in real time in the interest area. The surgeon is informed about the activity of the investigated site both through the direct visualization of the number of the detected photons and through a sound indicator, modulated in frequency in a way proportional to the size of the counting itself.

The known probes detect the incident radiations when they are brought near the source, so that the sanitary operator can identify the lymph node thereon he/she has to intervene.

In its most simplified form, then the examination consists in a sweeping performed with the probe head on the whole area wherein the lymph node could be localized.

However, in this way, the time needed to identify the lymph node can be long, by increasing the risk of complications for the patient which increase proportionally to the length of the surgical operation.

In order to obviate this drawback, one has thought to obtain a partial image of a patient subjected to this examination, with imaging techniques and instruments designated as gamma chamber with reduced sizes.

Even with these stratagems, the procedure requests long time due to the minor efficiency of these detection apparatuses.

Therefore, it has been proposed to combine the use of a scintigraphic probe with imaging techniques in order to obtain substantially a navigation system of the probe able to guide it in a more direct way to the source direction.

However, this combination results to be remarkably complex for instrumentation and implementation techniques.

The US patent Nr. 3,539,806 describes a scintigraphic probe for the detection of the emission of a radiation and its origin direction in order to identify a radiations source, and comprising:
- a first scintigraphic detecting element, comprising a substantially tubular body, said body being hollow, provided with a proximal opening, and divided in at least three longitudinal sectors each formed by a scintillation crystal of the same type;
- a second scintigraphic detecting element comprising a scintillation crystal internally housed in said tubular body such that it is laterally shielded and having an un-shielded surface in correspondence of said proximal opening; and
- photodetecting means associated to said scintillation crystals.

The technical problem underlying the present invention is to provide a scintigraphic probe allowing to obviate the drawbacks mentioned with reference to the known art.

The idea of solution consists in a goniometric probe, thus able to provide directly to the operator indications related not only to the position, but even to the direction to be followed to reach the radiant source.

Such problem is therefore solved by a probe as above specified, comprising:
- a first scintigraphic detecting element, comprising a substantially tubular body, said body being hollow and provided with a proximal opening, divided at least into three longitudinal sectors each formed by a scintillation crystal having a respective scintillation and/or light collection feature different from the other ones;
- a second scintigraphic detecting element comprising a scintillation crystal housed inside said tubular structure so as to be laterally shielded thereby and having an unschielded surface at said proximal opening; and
- photo-detecting means coupled to the above-mentioned scintillation crystals.

The main advantage of the scintigraphic goniometric probe according the present invention lies in the fact of allowing a quick identification of the radiant source in a scintigraphic examination by using a simple instrument for the operator and with small sizes, even for the simplification of the photo-detecting means needed for the probe itself.

The detection system constituted by the probe defined above is then particularly suitable to detect the sentinel lymph nodes using the technique of the local administration of radioactive solutions or radiomedicines near the tumour.

In particular, the system results to be suitable for the intra-surgical use, wherein localization rapidity and precision are a fundamental requirement.

The so-constructed probe offers a high sensibility and efficiency for all other intra-surgical operations involving the localization of tumoral tissues or lesions showing a high specificity to the radiomedicine, in particular for the applications of radio-guided surgery.

Scintigraphic applications linked however to the *in vivo* localization of concentrations of a radiomedicine in a human body are even possible, in order to localize them quickly with the purpose, for example, of a biopsy or a needle-biopsy.

Obviously, alternative probe uses are possible, in order to identify any radioactive source, for example with the purpose of higher safety in sensible areas such as the airport areas or in thermonuclear plants or in sites with radioactive contamination risk, even to detect radioactive waste disposed in a not correct or improper way.

The present invention will be described hereinafter according to a preferred embodiment thereof, provided by way of example and not with limitative purpose by referring to the enclosed drawings, wherein:

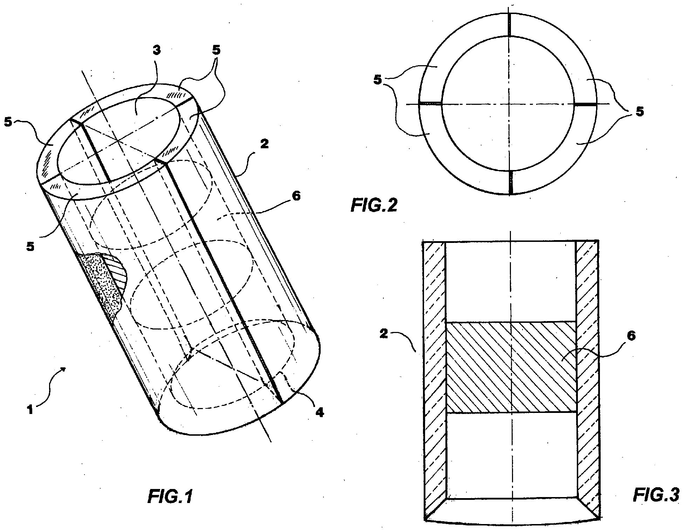

- figure 1 shows a perspective view, and in partial section, of a head of a scintigraphic goniometric probe according to the invention;
- figure 2 shows a plan view of the scintigraphic head of figure 1; and
- figure 3 shows a view in longitudinal section of the scintigraphic head of figure 1.

By referring to the figures, a scintigraphic probe head is designated as a whole with 1. It encloses the scintillation elements detecting the existence of a source of ionizing radiations.

It comprises a first scintigraphic detection element which is formed by a substantially tubular and hollow structure 2, so as to have a front proximal opening 3, which will be faced towards the area wherein the radioactive source is presumably localized, and a distal opening 4.

In the present embodiment, the tubular structure 2 has a cylindrical geometry with circular section. It is divided at least in three longitudinal sectors 5 which, in the present example, are four and they have the same angular extension of 90°.

Each sector 5 is constituted by a respective scintillation crystal with a respective scintillation feature.

This scintillation crystal is of the type with high atomic number. It can be implemented in Bismuth Germanate (BGO: Bi₄Ge₃O₁₂ or Bi₁₂GeO₂₂) or in Cerium-doped Lutetium oxyorthosilicate (LSO(Ce) Lu₂SiO₅:Ce) or in Cerium-doped Lutetium Yttrium orthosilicate (LYSO - Lu_{2(1-*x*)}Y_{2*x*}SiO₅:Ce,) which have a light emitting efficiency of about 12 and 30 photons/KeV (scintillation efficiency), in case of absorption of gamma radiation as by a radiomedicine containing Tc^{99m}, I¹³¹, In¹¹¹.

The crystal scintillation feature of each sector 5 has to be different from the other ones and this can be obtained by using crystals slightly different therebetween and with known scintillation properties.

Alternatively, or together with the above-mentioned effect, it is possible that the light collection feature varies from crystal to crystal. This effect can be obtained in many ways, for example by making that each crystal has different optical properties.

A way to provide a different optical property to each crystal can be that of applying thereto a coating characterized by a certain transmittance, so that each crystal has a treatment and a coating of the surfaces different from the other ones.

This allows modulating the photonic emissions produced by each crystal according to a characteristic intensity band, by allowing to recognize the emission of each crystal with a single photodetector, as it will appear in more details hereinafter in the description.

The tubular body 2, being constituted by crystals with a high atomic number, has the capability of shielding laterally the inside thereof.

It houses in its own cavity a second element of scintigraphic detection 6 comprising a scintillation crystal, laterally shielded by the tubular body 2.

Therefore, it has a not shielded sensible surface at said proximal opening 3.

In order to avoid that the second element 6 is reached by radiations coming laterally, then transversal to the longitudinal axis of the tubular body, it is placed in a position intermediate to the tubular body 2, so as to release a body front proximal portion and a distal portion.

The second element 6 occupies all room assigned thereto and therefore it has a cylindrical shape with diameter substantially equal to that of the cavity housing it.

In order to safeguard the correct operation thereof, all crystals under consideration, then the longitudinal sectors 5 and the second element 6, have to be insulated optically therebetween by specific coatings.

The scintillation crystal of the second element 6 advantageously could be a Cerium-doped Lanthanum Bromide (LaBr₃:Ce), which has a light emitting efficiency of 65 photons/KeV, or a Thallium-doped Sodium Iodide (NaI(Tl), which has a light emitting efficiency of 38 photons /KeV, or Thallium-doped or Sodium-doped Caesium Iodide (CsI(Tl),CsI(Na)) with a light emitting efficiency of 52 and 38 photons /keV, respectively. Therefore, all these crystals have a high light emitting efficiency.

Below the probe head photo-detecting means is placed, coupled to the above-mentioned scintillation crystals, which receive the photons produced thereby.

They comprise photo detectors substantially of conventional type, operating according to the electron multiplication principle or semiconductors (SD Silicon Detectors, SDD Silicon Detectors, APD Avalanche Silicon Detectors and SiPM, Silicon electron multiplier based upon Geiger discharge).

Such detectors can have, or not, position detection features, due to the more sophisticated and precise role in localizing multiple sources existing in the radiation field.

However, the crystal differentiation of the first element and the substantial crystal diversity of the second element allow the signals obtainable through the photonic emission to be on different intensity bands.

Therefore, a specific software can distinguish, in case of the first detection element, which one of the crystals emits photons and to which extent, thus by providing a vectorial indication of the origin direction of the ionizing radiation.

In this way, the probe has the peculiarity of detecting the radiation origin/emission even in absence of defining the radiation detection direction contrary to many imaging apparatuses such as the scintigraphic gamma chambers.

The scintigraphic goniometric probe then comprises the above-described head.

It does not request additional components except a handle of conventional type and the connections between photodetecting means and a processor including the processing and goniometric pointing software.

Therefore, it can be easily handled and, for weight and volume, it may be contained in one hand.

The above-described head could have a whole diameter comprised between 10 mm and 20 mm, with a thickness of the tubular body from 2 to 4 mm and a height of about 50 mm.

The inner crystal height could be, for example, 5 ÷ 15 mm so that the front and rear free portions have a height of 10 ÷ 20 mm.

The inner detector will have the aim of detecting the incident radiation parallel to the head axis and it activates then only when the probe is faced to the source, by providing an energy photopeak comprised between 70 and 360 keV.

The use method consists in positioning the probe in a generic position of the observation field. After some detection seconds, photopeak events will be accumulated by four or more crystals of the hollow tubular body so as to process the radiation direction with a value comprised between 0°-360°.

Under the guide of a navigator and of specific visual and/or sound signals which will show up, down, right, left, the operator will move the system until arriving onto the position wherein the detector is in front of the source.

During the phases for approaching the source, the related variation of the countings with the distance square opposite will allow to provide even the remaining distance and the final coordinates of the source itself.

The data coming from the two inner and outer scintillation cylinders will allow calculating the best centering position and will allow the navigation system to establish the exact position of the radioactive source.

The so-constructed apparatus can provide the very high counting rates, analogously to existing apparatuses which however do not provide information about the radiation origin direction and at least 100 times higher than imaging systems such as small gamma chambers for scintigraphy.

With proper use manual skill and navigation ability, the system is able to detect radiation sources with a precision up to 3 mm, with so high efficiencies to succeed in localizing them in a period of time variable between second fractions and few tens of seconds, depending upon the vision field sizes and the radioactivity existing in each single source.

A so-constructed probe could constitute even a direction guiding system connected to a telecamera which thus can make to localize visually even in a dynamic way a radioactive object (a person, a moving suitcase).

In case of fixed apparatuses, a triangulation system based upon three apparatuses positioned in different places can result to be particularly efficient.

To the above-described scintigraphic goniometric probe a person skilled in the art, in order to satisfy additional and contingent needs, could introduce several additional modifications and variants, all however within the protective scope of the present invention, as defined by the enclosed claims.

## Claims

1. A scintigraphic goniometric probe, for the detection of the emission of a radiation and its origin direction in order to identify a radiations source, in particular during a scintigraphic examination, comprising:
• a first scintigraphic detecting element, comprising a substantially tubular body (2), said body being hollow, provided with a proximal opening, and divided in at least three longitudinal sectors (5) each formed by a scintillation crystal having a respective scintillation and/or light transmission characteristic different from the others;
• a second scintigraphic detecting element (6) comprising a scintillation crystal internally housed in said tubular body (2) such that it is laterally shielded and having an un-shielded surface in correspondence of said proximal opening (3); and
• photo-detecting means associated to said scintillation crystals.

2. The probe according to claim 1, wherein the substantially tubular body is divided in four longitudinal sectors (5).

3. The probe according to claim 1, wherein the tubular body (2) is cylindrically shaped with a circular section, said longitudinal sectors (5) having equal angular extension.

4. The probe according to claim 1, wherein the tubular body (2) is formed by crystals of the same type, each with a surface treatment and providing different optical properties.

5. The probe according to claim 4, wherein said coating has different colours.

6. The probe according to claim 1, wherein the tubular body (2) is formed by crystals with high atomic numbers, in particular formed by a crystal selected from the group consisting of: Bismuth Germanate (BGO: Bi₄Ge₃O₁₂ or Bi₁₂GeO₂₂), Cerium-doped Lutetium oxyorthosilicate (LSO(Ce) Lu2SiO5:Ce), Cerium-doped Lutetium Yttrium orthosilicate (LYSO Lu_{2(1-*x*)}Y_{2*x*}SiO₅:Ce,).

7. The probe according to claim 1, wherein the second element (6) is placed in a tubular body (2) intermediate position, so as to release a body proximal front portion and a distal portion, for preventing it to be reached by lateral radiations, i.e. transversal to the tubular body (2) longitudinal axis.

8. The probe according to claim 1, wherein scintillation crystals of the longitudinal sectors (5) of the second element (6) are optically insulated between them by suitable coatings.

9. The probe according to claim 1, wherein the scintillation crystal del second element (6) is a crystal selected from the group consisting of: Cerium-doped Lanthanum Bromide (LaBr₃:Ce), Thallium-doped Sodium Iodide (NaI(Tl)), Thallium or Sodium-doped Cesium Iodide (CsI(Tl),CsI(Na)).

10. The probe according to claim 1, wherein the photo-detecting means operates according to electron multiplication principle.

11. The probe according to claim 1, wherein the photo-detecting means are semiconductors selected from the group consisting of SD Silicon Detectors, SDD Silicon Drift Detectors, APD Avalanche Silicon Detectors and SiPM, Silicon photomultipliers based on Geiger discharge.

12. The probe according to claim 1, comprising a navigation system providing up, down, right and left signals.

13. A direction guiding system connected to a video camera comprising a scintigraphic probe as any of the preceding claims.

## Patentansprüche

1. Szintigraphische goniometrische Sonde zur Detektion der Emission einer Strahlung und deren Ursprungsrichtung, um eine Strahlungsquelle, insbesondere während einer szintigraphischen Untersuchung zu identifizieren, umfassend:
- ein erstes szintigraphisches Detektionselement, umfassend einen im Wesentlichen rohrförmigen Körper (2), wobei der Körper hohl ist, mit einer proximalen Öffnung versehen ist und in mindestens drei Längsbereiche (5) unterteilt ist, von denen jeder durch einen Szintillationskristall gebildet wird, der eine entsprechende Szintillation und/oder Lichttransmissionseigenschaft aufweist, die zu den anderen unterschiedlich ist,
- ein zweites szintigraphisches Detektionselement (6) umfassend einen Szintillationskristall, der im Inneren des rohrförmigen Körpers (2) so aufgenommen ist, dass er seitlich abgeschirmt ist und eine nicht-abgeschirmte Oberfläche in Übereinstimmung mit der proximalen Öffnung (3) aufweist; und
- Photo-Detektionsmittel, die den Szintillationskristallen zugeordnet sind.

2. Sonde nach Anspruch 1, wobei der im Wesentliche rohrförmige Körper in vier Längsbereiche (5) unterteilt ist.

3. Sonde nach Anspruch 1, wobei der rohrförmige Körper (2) zylindrisch geformt ist mit einem kreisförmigen Querschnitt aufweist, wobei die Längsbereiche (5) gleiche Winkelerstreckungen aufweisen.

4. Sonde nach Anspruch 1, wobei der rohrförmige Körper (2) durch Kristalle der gleichen Art gebildet ist, jeder mit einer Oberflächenbehandlung und wobei jeder unterschiedliche optische Eigenschaften liefert.

5. Sonde nach Anspruch 4, wobei die Beschichtung unterschiedliche Farben aufweist.

6. Sonde nach Anspruch 1, wobei der rohrförmige Körper (2) durch Kristalle mit hoher Ordnungszahl gebildet ist, insbesondere gebildet ist aus einem Kristall, der aus der Gruppe bestehend aus: Wismutgermanat (BGO: Bi₄Ge₃O₁₂ oder Bi₁₂GeO₂₂), Zer-dotiertes Lutetiumoxyorthosilikat (LSO(Ce) Lu₂SiO₅:Ce), Zer-dotiertes Lutetiumyttriumorthosilikat (LYSO Lu₂₍₁₋ₓ₎Y₂ₓSiO₅:Ce) ausgewählt ist.

7. Sonde nach Anspruch 1, wobei das zweite Element (6) in einer Zwischenposition des rohrförmigen Körpers (2) platziert ist, so dass ein proximaler Frontbereich des Körpers und ein distaler Bereich freigegeben wird, um zu verhindern, dass dieses durch seitliche Strahlungen, das heißt transversal zu der Längsachse des rohrförmigen Körpers (2), erreicht wird.

8. Sonde nach Anspruch 1, wobei Szintillationskristalle der Längsbereiche (5) des zweiten Elementes (6) untereinander optisch isoliert sind durch geeignete Beschichtungen.

9. Sonde nach Anspruch 1, wobei der Szintillationskristall des zweiten Elementes (6) ein Kristall ist, der aus der Gruppe bestehend aus: Zer-dotiertes Lanthanbromid (LaBr₃:Ce), Thalliumdotiertes Natriumiodid (Nal(Tl)), Thallium- oder Natrium-dotiertes Caesiumiodid (Csl(Tl), CsI(Na)) ausgewählt ist.

10. Sonde nach Anspruch 1, wobei das Photo-Detektionsmittel entsprechend dem Elektronenvervielfachungsprinzip arbeitet.

11. Sonde nach Anspruch 1, wobei die Photo-Detektionsmittel Halbleiter sind, die aus der Gruppe bestehend aus SD Silizium Detektoren, SDD Silizium Drift Detektoren, APD Avalanche Silizium Detektoren und SiPM, Silizium Photo-Multiplier basierend auf Geiger-Entladung ausgewählt sind.

12. Sonde nach Anspruch 1, umfassend ein Navigationssystem, das Hoch-, Runter-, Rechts- und Linkssignale liefert.

13. Richtungsführungssystem verbunden mit einer Videokamera umfassend eine szintigraphische Sonde nach einem der vorstehenden Ansprüche.

## Revendications

1. Sonde goniométrique scintigraphie, pour la détection de l'émission d'un rayonnement et de sa direction d'origine afin d'identifier une source de rayonnements, en particulier lors d'un examen scintigraphique, comprenant :
• un premier élément de détection scintigraphique, comprenant un corps sensiblement tubulaire (2), ledit corps étant creux, pourvu d'une ouverture proximale, et divisé en au moins trois secteurs longitudinaux (5) formés chacun par un cristal de scintillation ayant une caractéristique respective de scintillation et/ou de transmission de la lumière différente des autres ;
• un second élément de détection scintigraphique (6) comprenant un cristal de scintillation logé à l'intérieur dudit corps tubulaire (2) de façon à ce qu'il soit protégé latéralement et ayant une surface non protégée en correspondance avec ladite ouverture proximale (3) ; et
• des moyens de photodétection associés auxdits cristaux de scintillation.

2. Sonde selon la revendication 1, dans laquelle le corps sensiblement tubulaire est divisé en quatre secteurs longitudinaux (5).

3. Sonde selon la revendication 1, dans laquelle le corps tubulaire (2) a une forme cylindrique avec une section circulaire, lesdits secteurs longitudinaux (5) présentant une extension angulaire égale.

4. Sonde selon la revendication 1, dans laquelle le corps tubulaire (2) est formé par des cristaux de même type, chacun ayant un traitement de surface et fournissant propriétés optiques différentes.

5. Sonde selon la revendication 4, dans laquelle ledit revêtement a différentes couleurs.

6. Sonde selon la revendication 1, dans laquelle le corps tubulaire (2) est formé par des cristaux ayant des numéros atomiques élevés, en particulier formé par un cristal choisi dans le groupe constitué : d'un germanate de bismuth (BGO : Bi₄Ge₃O₁₂ ou Bi₁₂GeO₂₂), d'un oxyorthosilicate de lutétium dopé au cérium (LSO(Ce) Lu2SiO5:Ce), d'un orthosilicate de lutétium et d'yttrium dopé au cérium (LYSO Lu₂₍₁₋ₓ₎Y₂ₓSiO₅:Ce).

7. Sonde selon la revendication 1, dans laquelle le second élément (6) est placé à une position intermédiaire du corps tubulaire (2), afin de libérer une partie avant proximale et une partie distale de corps, pour éviter qu'il soit atteint par des rayonnements latéraux, c'est-à-dire transversaux par rapport à l'axe longitudinal du corps tubulaire (2).

8. Sonde selon la revendication 1, dans laquelle les cristaux de scintillation des secteurs longitudinaux (5) du second élément (6) sont optiquement isolés entre eux par des revêtements appropriés.

9. Sonde selon la revendication 1, dans laquelle le cristal de scintillation del second élément (6) est un cristal choisi dans le groupe constitué : d'un bromure de lanthane dopé au cérium (LaBr₃:Ce), d'un iodure de sodium dopé au thallium (NaI(Tl)), d'un iodure de césium dopé au thallium ou sodium (CsI(Tl), CsI(Na)).

10. Sonde selon la revendication 1, dans laquelle les moyens de photodétection fonctionnent selon le principe de la multiplication des électrons.

11. Sonde selon la revendication 1, dans laquelle les moyens de photodétection sont des semi-conducteurs choisis dans le groupe constitué des détecteurs au silicium SD, des détecteurs à dérive au silicium SDD, des détecteurs au silicium à avalanche APD et des photomultiplicateurs au silicium SiPM basés sur la décharge Geiger.

12. Sonde selon la revendication 1, comprenant un système de navigation fournissant des signaux haut, bas, droite et gauche.

13. Système de guidage de direction connecté à une caméra vidéo comprenant une sonde scintigraphique selon l'une quelconque des revendications précédentes.
